# FASCICULE DE BREVET EUROPEEN

(11) **EP 3 355 872 B1**
(45) Date de publication et mention de la délivrance du brevet: **27.05.2020**
(21) Numéro de dépôt: 16790639.5
(22) Date de dépôt: 29.09.2016
(51) Int. Cl.: A61K 31/045, A61K 31/08, A61K 31/25, A61P 27/16

(54) **COMPOSITION DE NETTOYANT AURICULAIRE**
AURIKULÄRE REINIGUNGSZUSAMMENSETZUNG
AURICULAR CLEANING COMPOSITION

(30) Priorité: 30.09.2015 FR 1559298
(43) Date de publication de la demande: 08.08.2018
(73) Titulaire: Vetoquinol SA, 70200 Magny-Vernois (FR)
(72) Inventeur: MOREAU, Marinette, 70200 Saint-Germain (FR); BREVET, Aurélie, 70200 Lure (FR); LEGO, Elodie, 70200 Lure (FR)
(74) Mandataire: Novagraaf Technologies
(86) Numéro de dépôt international: PCT/FR2016/052496
(87) Numéro de publication internationale: WO 2017/055762

(56) Documents cités:
- US-A1- 2009 318 558
- DATABASE MEDLINE [Online] US NATIONAL LIBRARY OF MEDICINE (NLM), BETHESDA, MD, US; mars 2012 (2012-03), SILVERSTEIN HERBERT ET AL: "A prospective study to evaluate the efficacy of isopropyl alcohol irrigations to prevent cerumen impaction.", XP002757634, Database accession no. NLM22430344 & SILVERSTEIN HERBERT ET AL: "A prospective study to evaluate the efficacy of isopropyl alcohol irrigations to prevent cerumen impaction.", EAR, NOSE, & THROAT JOURNAL MAR 2012, vol. 91, no. 3, mars 2012 (2012-03), pages E25-E28, ISSN: 1942-7522

## Description

### PRIORITÉ

La présente demande de brevet revendique la priorité de la demande de brevet français N° FR1559298 déposée le 30 septembre 2015.

### DESCRIPTION

### Domaine technique

Le domaine technique de la présente invention concerne les compositions nettoyantes auriculaires à usage humain ou animal.

La présente invention concerne plus précisément une composition de nettoyant auriculaire comprenant de l'alcool isopropylique et/ou de l'éther monoéthylique de diéthylène glycol en tant qu'agent(s) céruménolytique (s), éventuellement en combinaison avec du caprylcaproyl polyoxyl-8 glycéride. Dans la description ci-dessous, les références entre crochets [ ] renvoient à la liste des références présentée à la fin du texte.

### État de la technique

Les otites externes chez le chien sont une pathologie courante. Leur traitement nécessite une bonne connaissance de la physiopathologie de ces maladies afin de proposer un nettoyage spécifique selon l'examen clinique. De nombreux nettoyants auriculaires sont actuellement disponibles en France et à l'étranger. Ils contiennent plusieurs principes actifs et excipients, et ne nécessitent pas d'autorisation de mise sur le marché.

Le nettoyage auriculaire a pour but de retirer le cérumen en excès, pour rendre la surface de l'épiderme qui tapisse l'oreille externe accessible au traitement médical.

Les agents céruménolytiques sont couramment utilisés dans les nettoyants auriculaires pour dissoudre le cérumen. Il en existe deux types: les céruménolytiques au sens strict, qui rompent l'intégrité du cérumen en lysant l'agglomérat de squames, et les simples lubrifiants qui ont une action limitée au ramollissement et à l'évacuation du cérumen. Un céruménolytique est censé rompre la liaison entre les cornéocytes, et ainsi ramollir, liquéfier, et/ou dissoudre le cérumen. Il fragilise la membrane des cornéocytes et permet, grâce à un gradient osmotique, le passage de l'eau et la fragmentation de ces cellules. Leur but est aussi de rompre les adhérences entre le bouchon et la paroi du canal (Hawke, 2002 [1]).

Le docusate sodique, le bicarbonate de sodium, l'eau oxygénée, et le peroxyde d'urée sont des produits céruménolytiques utilisés couramment en médecine humaine (Malard *et al.* 2005 **[2]**). Le peroxyde de carbamide, le polysorbate 80, le laurylsulfate de sodium et le xylène sont aussi des molécules avec des propriétés céruménolytiques.

En outre, certains autres agents ont été rapportés pour être quelque peu efficaces dans le ramollissement de la cire d'oreille. De tels agents comprennent la glycérine (glycérol), l'huile d'olive, l'huile d'amande, l'huile minérale, le carbonate de sodium, et le dichlorobenzène. Après ramollissement avec un de ces agents, l'irrigation avec de l'eau à la température du corps ou de sérum physiologique est souvent effectuée pour enlever le cérumen ramolli. Le dichlorobenzène résulte parfois en une irritation du conduit auditif.

L'utilisation d'isopropanol à 70% comme solution de rinçage du canal auditif externe chez l'homme pour réduire l'accumulation du cérumen a également été rapportée (cf. « A prospective study to evaluate the efficacy of isopropyl alcohol irrigations to prevent cerumen impaction » EAR, NOSE & THROAT JOURNAL, 2012, vol. 91(3), pp. E25-E28).

Des compositions qui facilitent le nettoyage de la cire d'oreille ont également fait l'objet de plusieurs brevets. Par exemple, le brevet U.S. 3 422 186 (Sasmor, **[3]**) décrit des compositions céruménolytiques comprenant des condensats d'éthylène oxyde -polyoxypropylène glycol. Le brevet U.S. 4 895 875 (Winston, **[4]**) décrit des solutions de peroxyde stabilisées comprenant du peroxyde d'urée et de la glycérine, et leur utilisation pour l'élimination du cérumen. Le brevet U.S. 5 296 472 (Sanchez et al, **[5]**) décrit des compositions comprenant des cyclodextrines et des méthodes d'utilisation pour l'élimination du cérumen. Le brevet U.S. 5 380 711 (Sanchez et al, **[6]**) décrit des compositions sans huile à base de cyclodextrines "vides" et méthodes d'utilisation pour l'élimination du cérumen. Et le brevet U.S. 5 480 658 (Melman, **[7]**) décrit des compositions aqueuses comprenant de l'acide acétique et de l'acide borique pour le nettoyage du conduit auditif externe d'animaux domestiques.

Une solution aqueuse de bicarbonate de sodium à 5% est souvent préparée et utilisée par des médecins pour traiter les bouchons de cérumen. Cette solution peut être préparée avec ou sans glycérine. Cependant, ces solutions ne sont pas stables, et de ce fait, des solutions de bicarbonate de sodium n'ont jamais été mises au point dans des produits commerciaux « prêts à l'emploi ».

Il existe donc un besoin pour une composition améliorée permettant d'éliminer efficacement le cérumen, qui soit commercialement viable et qui ne souffre pas des limitations des céruménolytiques actuellement disponibles.

### EXPOSÉ DE L'INVENTION

La présente invention a précisément pour but de répondre à ces besoins et inconvénients de l'art antérieur en fournissant une composition auriculaire nettoyante, comprenant :
- de l'alcool isopropylique et/ou de l'éther monoéthylique de diéthylène glycol en tant qu'agent(s) céruménolytique(s), éventuellement en combinaison avec du caprylcaproyl polyoxyl-8 glycéride ; et
- éventuellement un véhicule aqueux otologiquement acceptable.

Il existe deux types de solutions céruménolytiques : les solutions aqueuses et les solutions huileuses. Les solutions aqueuses peuvent être préférées aux solutions huileuses pour deux raisons. D'abord, les solutions aqueuses sont évacuées plus facilement du conduit et permettent donc un nettoyage et un séchage plus rapide. De plus, les solutions huileuses forment un film occlusif et, si elles ne sont pas bien évacuées, favorisent le développement des bactéries. Ainsi, avantageusement, la composition auriculaire nettoyante selon l'invention est une solution non huileuse, et contient un véhicule aqueux.

La composition peut contenir tout excipient utilisé conventionnellement dans les compositions nettoyantes auriculaires, tels que:
- les agents asséchants qui assèchent la surface du conduit auditif afin de prévenir le phénomène de macération pouvant favoriser la prolifération bactérienne.
- les anti-séborrhéiques
- des émollients
- des antiseptiques
- des anti-inflammatoires ou agents apaisants
- des parfums etc...

Ainsi, la composition selon l'invention peut comprendre en outre un agent anti-séborrhéique, un émollient, un tensioactif, un agent tampon, un agent anti-septique, un agent anti-inflammatoire (ou agent apaisant) et/ou un conservateur.

Parmi les agents anti-séborrhéiques utilisables dans le contexte de l'invention, on peut citer par exemple les lipoaminoacides tels que le capryloyl glycine (« Lipacid C8G® ») ou l'undécylénoyl glycine (« Lipacid UG® »), ou un mélange de ceux-ci.

Parmi les émollients utilisables dans le contexte de l'invention, on peut mentionner par exemple la glycérine, le propylèneglycol, le polyéthylèneglycol, la triacétine, les dérivés siliconés (ex : diméthicone), les polyols ou un mélange d'au moins deux de ceux-ci.

Parmi les tensioactifs utilisables dans le contexte de l'invention, on peut mentionner par exemple les polysorbates tels que le monooléate de sorbitane polyoxyéthylène, les polymères de 4-(1,1,3,3-tétraméthylbutyl)phénol/poly(oxyéthylène), les copolymères séquencés de poly(oxyéthylène)-poly(oxypropylène), les esters de polyéthylèneglycol d'acides gras, les éthers de polyoxypropylène d'alcanes en C₁₂ à C₁₈, ou un mélange d'au moins deux de ceux-ci. De préférence, il pourra s'agir de polysorbates tels que le monooléate de sorbitane polyoxyéthylène, les copolymères séquencés de poly(oxyéthylène)-poly(oxypropylène), les esters de polyéthylèneglycol d'acides gras, les éthers de polyoxypropylène d'alcanes en C₁₂ à C₁₈, ou un mélange d'au moins deux de ceux-ci.

Parmi les agents tampon utilisables dans le contexte de l'invention, on peut mentionner par exemple le trishydroxyméthylaminométhane (ou 2-Amino-2-hydroxymethyl-propane-1,3-diol, ou « trométhamine »)), le citrate, le phosphate, le borate, l'acétate, l'hydroxyde de sodium, la glycine, la triéthanolamine, les sels de l'un quelconque de ceux-ci, ou un mélange d'au moins deux de ceux-ci.

Dans la présente, on entend par "borate" ou « acide borique » un composé inorganique comprenant du bore et un ou plusieurs groupes d'oxygène, et qui est soit sous forme acide ou basique lorsqu'il est dissous dans une composition de la présente invention. Les sources de composés borate / acide borique comprennent les sels de métaux alcalins de borate, l'acide borique et le borax. Le composé borate / acide borique peut également contribuer à la préservation anti-microbienne des compositions de la présente invention à un niveau efficace pour la distribution multi-usage. La solubilité des composés borate / acide borique dans l'eau peut être limitée. Cependant, la solubilité de ces composés peut être augmentée en ajoutant par exemple un polyol monomérique ou polymérique.

Dans la présente, on entend par "polyol monomérique" un composé ayant 2 à 6 atomes de carbone et au moins deux groupes hydroxy. Des exemples de polyols monomériques incluent la glycérine, le propylène glycol, l'éthylène glycol, le sorbitol et le mannitol. De préférence, les polyols monomériques sont choisis parmi les polyols ayant 2-3 atomes de carbone et au moins deux groupes hydroxyle, tels que la glycérine, le 1,2-propane diol ("le propylène glycol"), le 1,3-propane diol et de l'éthylène glycol. Par exemple, il peut s'agir de la glycérine.

Dans la présente, on entend par "polyol polymérique" un glycol polyalcoxylé ayant une masse moléculaire allant d'environ 200 à 600 Daltons. Des exemples de polyols polymériques incluent le polyéthylène glycol 200 (désignant un poids moléculaire de 200 daltons, "PEG 200") et le PEG 400. Le PEG peut éventuellement être monoalcoxylé. Des exemples de PEG monoalcoxylés comprennent le monométhoxy PEG 200 et éthoxy PEG 400. Bien que ces PEG alcoxylés ne soient pas techniquement des polyols, ils sont de structure similaire aux PEG non-alkoxylatés. Par conséquent, aux fins de la définition, ils sont inclus dans l'expression « polyol polymérique ».

Parmi les agents anti-septiques utilisables dans le contexte de l'invention, on peut mentionner par exemple un extrait de calendula (le souci) et les anti-septiques de la famille des biguanides telle que la chlorhexidine, ou un mélange d'au moins deux de ceux-ci.

Parmi les agents anti-inflammatoires (ou agents apaisants) utilisables dans le contexte de l'invention, on peut mentionner par exemple un extrait de calendula (le souci).

Parmi les conservateurs utilisables dans le contexte de l'invention, on peut mentionner par exemple l'acide borique, le dichlorure de poly [diméthylimino-2-butène-1,4-diyle]chlorure-alpha-[4-tris (2-hydroxyéthyl) ammonium], les halogénures de benzalkonium, les sels d'alexidine, les sels de chlorhexidine, ou un mélange d'au moins deux de ceux-ci.

Avantageusement, la composition selon l'invention peut comprendre de l'éther monoéthylique de diéthylène glycol en tant qu'agent céruménolytique. Celui-ci peut être utilisé comme seul agent céruménolytique dans la composition. Alternativement, l'éther monoéthylique de diéthylène glycol peut être utilisé en combinaison avec de l'alcool isopropylique et/ou du caprylcaproyl polyoxyl-8 glycéride.

Par exemple, la composition selon l'invention peut comprendre de l'éther monoéthylique de diéthylène glycol en tant qu'agent céruménolytique, en combinaison avec du caprylcaproyl polyoxyl-8 glycéride ; avec une teneur en éther monoéthylique de diéthylène glycol allant de 10 à 40% en poids, et une teneur en caprylcaproyl polyoxyl-8 glycéride allant de 20 à 60% en poids ; la teneur totale en éther monoéthylique de diéthylène glycol et caprylcaproyl polyoxyl-8 glycéride allant de 50 à 70% en poids ; les % étant exprimés par rapport au poids total de la composition.

Alternativement, la composition selon l'invention peut comprendre de l'alcool isopropylique en tant qu'agent céruménolytique , en combinaison avec du caprylcaproyl polyoxyl-8 glycéride ; avec une teneur en alcool isopropylique allant de 5 à 20% en poids, et une teneur en caprylcaproyl polyoxyl-8 glycéride allant de 20 à 60% en poids ; la teneur totale en alcool isopropylique et caprylcaproyl polyoxyl-8 glycéride allant de 35 à 75% en poids ; les % étant exprimés par rapport au poids total de la composition.

Alternativement, la composition selon l'invention peut comprendre de l'éther monoéthylique de diéthylène glycol et de l'alcool isopropylique en tant qu'agents céruménolytiques; avec une teneur en éther monoéthylique de diéthylène glycol allant de 14 à 33% en poids, et une teneur en alcool isopropylique allant de 20 à 40% en poids ; la teneur totale en éther monoéthylique de diéthylène glycol et alcool isopropylique allant de 35 à 70% en poids ; les % étant exprimés par rapport au poids total de la composition.

Alternativement, la composition selon l'invention peut comprendre de l'éther monoéthylique de diéthylène glycol et de l'alcool isopropylique en tant qu'agents céruménolytiques, en combinaison avec du caprylcaproyl polyoxyl-8 glycéride. Avantageusement, la composition selon l'invention peut comprendre une teneur en éther monoéthylique de diéthylène glycol allant de 25 à 35% en poids, une teneur en alcool isopropylique allant de 5 à 10% en poids, et une teneur en caprylcaproyl polyoxyl-8 glycéride allant de 20 à 35% en poids; la teneur totale en éther monoéthylique de diéthylène glycol, alcool isopropylique et caprylcaproyl polyoxyl-8 glycéride allant de 60 à 70% en poids ; les % étant exprimés par rapport au poids total de la composition.

Selon un autre aspect, l'invention concerne un procédé pour éliminer totalement ou en partie le cérumen, comprenant une étape consistant à introduire dans le canal externe de l'oreille d'un animal, l'une quelconque des compositions auriculaires nettoyantes selon l'invention décrites dans le présent document. L'apparition qde cérumen dans l'oreille est un phénomène normal (ce n'est pas une maladie ou une pathologie). L'introduction d'une composition selon l'invention dans le canal externe de l'oreille d'un sujet sain ne fait que nettoyer la paroi du canal sans aucun effet thérapeutique (la santé du sujet humain ou animal ne s'en trouve pas améliorée). Il s'agit donc d'un un procédé non thérapeutique pour éliminer totalement ou en partie le cérumen.

L'animal peut être un mammifère, de préférence un animal domestique tel que le chien ou le chat.

Les inventeurs ont découvert que le transcutol® (2-(2-Ethoxyéthoxy)éthanol, ou Ethoxydiglycol, ou éther monoéthylique de diéthylène glycol) avait un effet céruménolytique , et que cet effet était potentialisé avec du Labrasol® (du caprylcaproyl polyoxyl-8 glycéride).

Les inventeurs ont également découvert que l'effet céruménolytique de l'alcool isopropylique était également potentialisé avec du Labrasol® (du caprylcaproyl polyoxyl-8 glycéride).

Ainsi, selon un autre aspect, l'invention concerne l'utilisation d'une association d'au moins deux solvants sélectionnés parmi l'alcool isopropylique, l'éther monoéthylique de diéthylène glycol et le caprylcaproyl polyoxyl-8 glycéride pour son utilisation comme agent céruménolytique. De la même manière que pour le procédé d'élimination de cérumen précité, il s'agit d'une utilisation non thérapeutique, la production de cérumen dans l'oreille de l'homme ou de l'animal étant un phénomène normal (non pathologique).

Selon un autre aspect, l'invention concerne l'utilisation d'une association d'au moins deux solvants sélectionnés parmi l'alcool isopropylique, l'éther monoéthylique de diéthylène glycol et le caprylcaproyl polyoxyl-8 glycéride dans la préparation d'une composition destinée à éliminer totalement ou en partie le cérumen, en particulier chez l'animal.

Selon un autre aspect, l'invention concerne l'utilisation du caprylcaproyl polyoxyl-8 glycéride en tant qu'agent potentialisateur de l'effet céruménolytique de l'alcool isopropylique et/ou de l'éther monoéthylique de diéthylène glycol d'une composition auriculaire nettoyante. Ainsi, selon un aspect, l'invention concerne un procédé pour potentialiser l'effet céruménolytique de l'alcool isopropylique et/ou de l'éther monoéthylique de diéthylène glycol d'une composition auriculaire nettoyante, comprenant l'ajout d'une quantité efficace de caprylcaproyl polyoxyl-8 glycéride à la composition.

Tout particulièrement, la présente invention concerne l'utilisation de l'association de l'alcool isopropylique et/ou de l'éther monoéthylique de diéthylène glycol comme agent(s) céruménolytique (s), avec le caprylcaproyl polyoxyl-8 glycéride. Ainsi, selon un aspect, l'invention concerne un procédé pour éliminer totalement ou en partie le cérumen, comprenant une étape consistant à introduire dans le canal externe de l'oreille d'un sujet humain ou animal, une composition associant l'alcool isopropylique et/ou de l'éther monoéthylique de diéthylène glycol comme agent(s) céruménolytique (s), avec le caprylcaproyl polyoxyl-8 glycéride.

La présente invention concerne également l'utilisation de l'association de l'alcool isopropylique avec l'éther monoéthylique de diéthylène glycol comme agents céruménolytiques. Ainsi, la présente invention concerne également un procédé pour éliminer totalement ou en partie le cérumen, comprenant une étape consistant à introduire dans le canal externe de l'oreille d'un sujet humain ou animal, une composition associant l'alcool isopropylique avec l'éther monoéthylique de diéthylène glycol comme agents céruménolytiques.

La présente invention concerne également l'utilisation de la tri-association de l'alcool isopropylique avec l'éther monoéthylique de diéthylène glycol comme agent(s) céruménolytique (s), et le caprylcaproyl polyoxyl-8 glycéride. Ainsi, la présente invention concerne également une composition auriculaire nettoyante comprenant la tri-association de l'alcool isopropylique avec l'éther monoéthylique de diéthylène glycol comme agents céruménolytiques, an combinaison avec le caprylcaproyl polyoxyl-8 glycéride.

D'autres avantages pourront encore apparaître à l'homme du métier à la lecture des exemples ci-dessous, en référence aux figures annexées, donnés à titre illustratif, et non limitatif.

### BRÈVE DESCRIPTION DES FIGURES

**Figure 1** : Graphe représentant l'effet céruménolytique des matières premières utilisées seules (alcool isopropylique, Transcutol® et Labrasol®) :

| | |
|---|---|
| **1** | 100% Alcool isopropylique |
| **2** | 100% Transcutol® |
| **3** | 100% Labrasol® (lot 140734) |

**Figure 2** : Graphe représentant l'effet céruménolytique des compositions de nettoyant auriculaire basées sur l'association Transcutol®/Labrasol® :

| | |
|---|---|
| **1** | 140811: 30% Transcutol®, 40% Labrasol®, 2% Tween 80®, 17,0 % Eau, 1% Lipacide C8G®,1% Lipacide UG®,1% Trométhamine, 5% Glycérine, 3% Calendula |
| **2** | 140812: 30% Transcutol®, 30% Labrasol®, 2% Tween 80®,27,0 % Eau, 1% Lipacide C8G®,1% Lipacide UG®,1% Trométhamine, 5% Glycérine, 3% Calendula |
| **3** | 140813: 30% Transcutol®, 20% Labrasol®, 2% Tween 80®,37,0 % Eau, 1% Lipacide C8G®,1% Lipacide UG®,1% Trométhamine, 5% Glycérine, 3% Calendula |
| **4** | 140814: 20% Transcutol®, 50% Labrasol®, 2% Tween 80®,17,0 % Eau, 1% Lipacide C8G®,1% Lipacide UG®,1% Trométhamine, 5% Glycérine, 3% Calendula |
| **5** | 140817: 40% Transcutol®, 30% Labrasol®, 2% Tween 80®,17,0 % Eau, 1% Lipacide C8G®,1% Lipacide UG®,1% Trométhamine, 5% Glycérine, 3% Calendula |
| **6** | 140820: 10% Transcutol®, 60% Labrasol®, 2% Tween 80®,17,0 % Eau, 1% Lipacide C8G®,1% Lipacide UG®,1% Trométhamine, 5% Glycérine, 3% Calendula |

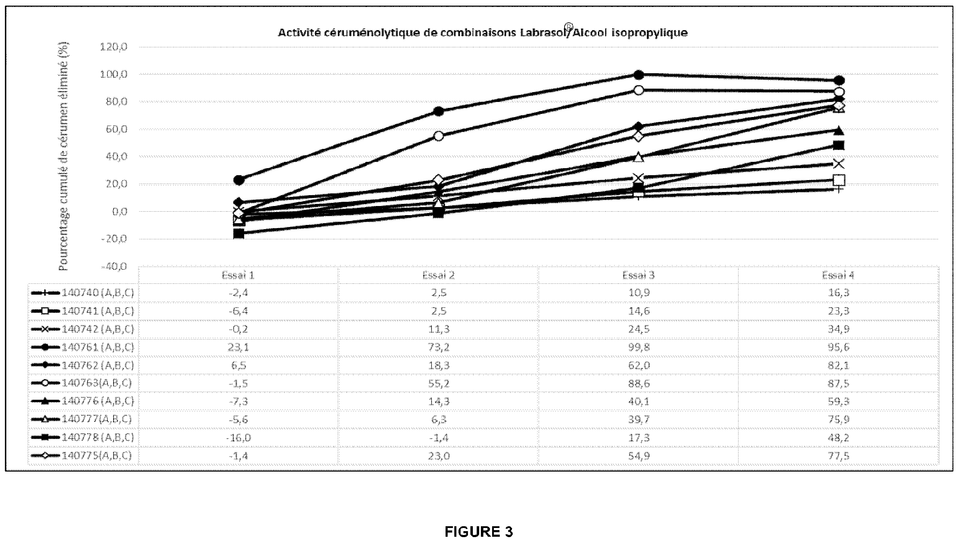

**Figure 3** : Graphe représentant l'effet céruménolytique des compositions de nettoyant auriculaire basées sur l'association alcool isopropylique/labrasol :

| | |
|---|---|
| **1** | 140740: 15% Alcool isopropylique, 20% Labrasol®,2% Tween 80®,51,50 % Eau, 1% Lipacide C8G®,1 % Lipacide UG®,1 % Trométhamine, 5% Glycérine, 3% Calendula, 0,5% parfum orange |
| **2** | 140741: 15% Alcool isopropylique, 30% Labrasol®,2% Tween 80®,41,50 % Eau, 1% Lipacide C8G®,1% Lipacide UG®,1 % Trométhamine, 5% Glycérine, 3% Calendula, 0,5% parfum orange |
| **3** | 140742: 15% Alcool isopropylique, 40% Labrasol®,2% Tween 80®,31,50 % Eau, 1% Lipacide C8G®,1% Lipacide UG®,1 % Trométhamine, 5% Glycérine, 3% Calendula, 0,5% parfum orange |
| **4** | 140761: 20% Alcool isopropylique, 40% Labrasol®,2% Tween 80®,26,50 % Eau, 1% Lipacide C8G®,1% Lipacide UG®,1 % Trométhamine, 5% Glycérine, 3% Calendula, 0,5% parfum orange |
| **5** | 140762: 15% Alcool isopropylique, 50% Labrasol®,2% Tween 80®,21,50 % Eau, 1% Lipacide C8G®,1% Lipacide UG®,1 % Trométhamine, 5% Glycérine, 3% Calendula, 0,5% parfum orange |
| **6** | 140763: 15% Alcool isopropylique, 60% Labrasol®,2% Tween 80®,11,50 % Eau, 1% Lipacide C8G®,1% Lipacide UG®,1 % Trométhamine, 5% Glycérine, 3% Calendula, 0,5% parfum orange |
| 7 | 140775: 10% Alcool isopropylique, 50% Labrasol®,2% Tween 80®,26,50 % Eau, 1% Lipacide C8G®,1% Lipacide UG®,1 % Trométhamine, 5% Glycérine, 3% Calendula, 0,5% parfum orange |
| 8 | 140776: 5% Alcool isopropylique, 50% Labrasol®,2% Tween 80®,31,50 % Eau, 1% Lipacide C8G®,1% Lipacide UG®,1 % Trométhamine, 5% Glycérine, 3% Calendula, 0,5% parfum orange |
| 9 | 140777: 10% Alcool isopropylique, 60% Labrasol®,2% Tween 80®,16,50 % Eau, 1% Lipacide C8G®,1% Lipacide UG®,1 % Trométhamine, 5% Glycérine, 3% Calendula, 0,5% parfum orange |
| 10 | 140778: 5% Alcool isopropylique, 60% Labrasol®,2% Tween 80®,21,50 % Eau, 1% Lipacide C8G®,1% Lipacide UG®,1 % Trométhamine, 5% Glycérine, 3% Calendula, 0,5% parfum orange |

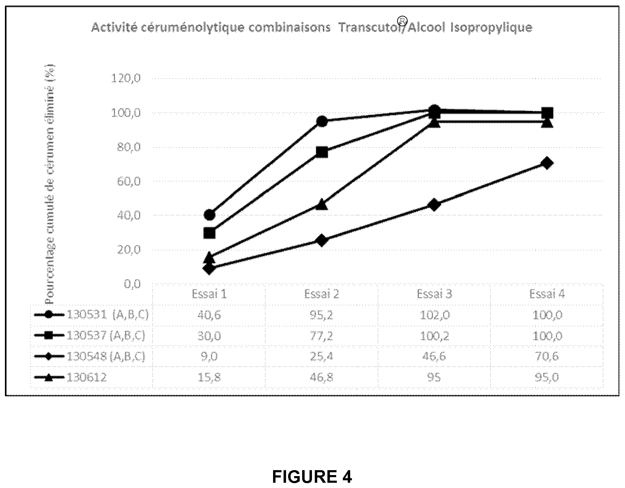

**Figure 4** : Graphe représentant l'effet céruménolytique des compositions de nettoyant auriculaire basées sur l'association Transcutol®/ alcool isopropylique :

| | |
|---|---|
| **1** | 130531 : 40% Alcool Isopropylique ; 30% Transcutol® ; 2% Tween 80®; 17% Eau; 1% Lipacide UG® ; 1% Lipacide C8G® ; 1% Trométhamine ; 5% glycérine ; 3% calendula |
| **2** | 130537 : 33% Alcool Isopropylique ; 22% Transcutol® ; 2% Tween 80®; 33% Eau; 1% Lipacide UG® ; 1% Lipacide C8G® ; 1% Trométhamine ; 5% glycérine ; 3% calendula |
| **3** | 130548 : 22% Alcool Isopropylique ; 14% Transcutol® ; 2% Tween 80®; 51% Eau; 1% Lipacide UG® ; 1% Lipacide C8G® ; 1% Trométhamine ; 5% glycérine ; 3% calendula |
| **4** | 130612 : 22% Alcool Isopropylique ; 33% Transcutol® ; 2% Tween 80®; 27% Eau; 1% Lipacide UG® ; 1% Lipacide C8G® ; 1% Trométhamine ; 10% glycérine ; 3% calendula |

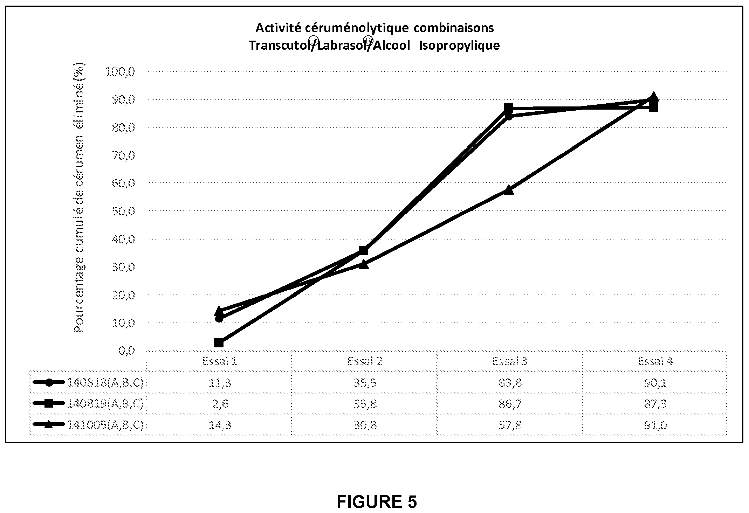

**Figure 5** **:** Graphe représentant l'effet céruménolytique des compositions de nettoyant auriculaire basées sur la tri-association Transcutol®/ alcool isopropylique/Labrasol® :

| | |
|---|---|
| **1** | 140818 : 30% Transcutol®, 35% Labrasol®, 5% Alcool Isopropylique , 2% Tween 80®,17,0 % Eau, 1% Lipacide C8G®,1% Lipacide UG®,1% Trométhamine, 5% Glycérine, 3% Calendula |
| **2** | 140819: 30% Transcutol®, 30% Labrasol®,10% Alcool Isopropylique , 2% Tween 80®,17,0 % Eau, 1% Lipacide C8G®,1% Lipacide UG®,1% Trométhamine, 5% Glycérine, 3% Calendula |
| **3** | 140105: 30% Transcutol®, 20% Labrasol®, 10% Alcool Isopropylique, 2% Tween 80®, 27% eau , 1% Lipacide C8G®,1% Lipacide UG®,1% Trométhamine, 5% Glycérine, 3% Calendula |

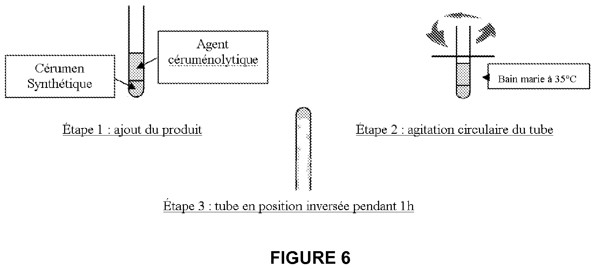

**Figure 6** **:** Illustration schématique d'un exemple de mise en œuvre du mode opératoire du test *in vitro* de l'effet céruménolytique, utilisé dans l'exemple 5.

Les exemples suivants sont donnés à titre illustratif et ne peuvent en aucun cas servir à limiter la portée de l'invention.

### EXEMPLES

Dans les exemples qui suivent, le terme « calendula » utilisé seul se réfère à un extrait de calendula commercialisé par la société Chemical&Cosmetic Company Finex (référence produit : ZN-02/FX-02, extrait de calendula dilué à 50% dans l'éthanol).

### Exemple 1 : Association Alcool Isopropylique/Labrasol®

Dans cet exemple, des compositions de nettoyant auriculaire ont été formulées à partir des composants suivants : alcool isopropylique, Labrasol®, Tween 80®, Eau, Lipacide C8G®, Lipacide UG®, Trométhamine, Glycérine, Calendula.

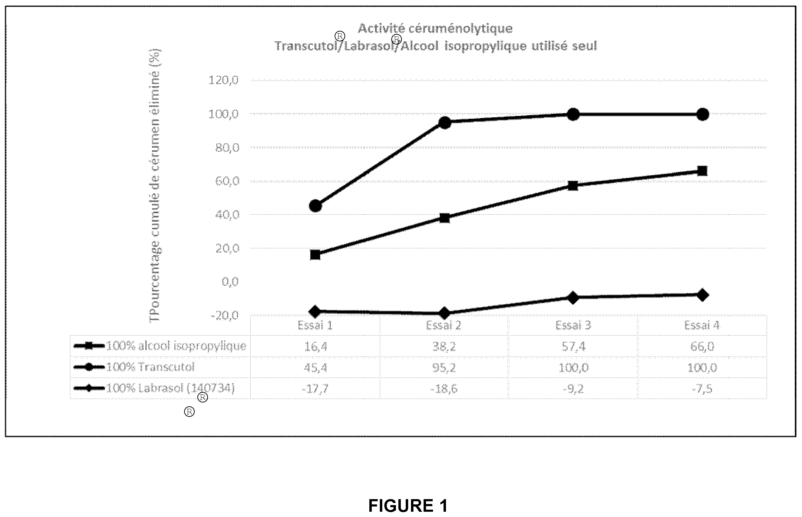

Les % en poids de chaque composant sont indiqués en Figure 1.

Le procédé utilisé est le suivant :

### Mélange 1

- Dans un contenant adapté, chauffer l'eau à 70°C puis ajouter dans l'ordre :
   - Lipacide C8G ®
   - Lipacide UG®
   - Trométhamine
- Agiter pendant 10 min à 600 rpm
- Refroidissement de la solution jusqu'à 40°C puis ajouter :
   - Tween 80 ®
- Agiter pendant 5 min à 600 rpm

### Mélange 2

- Incorporer dans un contenant adapté, et dans l'ordre :
   - Labrasol®
   - Alcool Isopropylique
- Agiter pendant 5 min à 600 rpm
- Ajouter le mélange 1
- Agiter pendant 5 min à 600 rpm
- Ajouter dans l'ordre :
   - Glycérine
   - Extrait de Calendula
- Agiter pendant 5 min à 600 rpm

### Exemple 2 : Association Transcutol®/Alcool Isopropylique :

Dans cet exemple, des compositions de nettoyant auriculaire ont été formulées à partir des composants suivants : Transcutol®, Alcool Isopropylique, Tween 80®, Eau, Lipacide C8G®, Lipacide UG®, Trométhamine, Glycérine, Calendula.

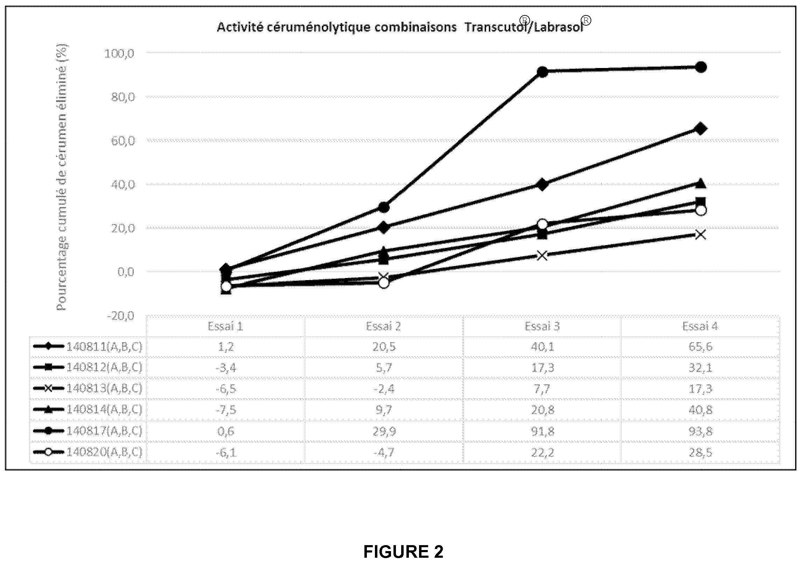

Les % en poids de chaque composant sont indiqués en Figure 2.

Le procédé utilisé est le suivant :
- Dans un contenant adapté, chauffer l'eau à 70°C puis ajouter dans l'ordre :
   - Lipacide C8G ®
   - Lipacide UG®
   - Trométhamine
- Agiter pendant 10 min à 600 rpm
- Refroidissement de la solution jusqu'à 40 °C puis ajouter :
   - Tween 80 ®
- Agiter pendant 5 min à 600 rpm
- Ajouter dans l'ordre :
   - Transcutol ®
   - Alcool Isopropylique
- Agiter pendant 5 min à 600 rpm
- Ajouter dans l'ordre :
   - Glycérine
   - Extrait de Calendula
- Agiter pendant 5 min à 600 rpm

### Exemple 3: Association Transcutol®/Labrasol®:

Dans cet exemple, des compositions de nettoyant auriculaire ont été formulées à partir des composants suivants : Transcutol®, Labrasol®, Tween 80®, Eau, Lipacide C8G®, Lipacide UG®, Trométhamine, Glycérine, Calendula.

Les % en poids de chaque composant sont indiqués en Figure 3.

Le procédé utilisé est le suivant :

### Mélange 1

- Dans un contenant adapté, chauffer l'eau à 70°C puis ajouter dans l'ordre :
   - Lipacide C8G ®
   - Lipacide UG®
   - Trométhamine
- Agiter pendant 10 min à 600 rpm
- Refroidissement de la solution jusqu'à 40 °C puis ajouter :
   - Tween 80 ®
- Agiter pendant 5 min à 600 rpm

### Mélange 2

- Incorporer dans un contenant adapté, et dans l'ordre :
   - Labrasol ®
   - Transcutol ®
- Agiter pendant 5 min à 600 rpm
- Ajouter le mélange 1
- Agiter pendant 5 min à 600 rpm
- Ajouter dans l'ordre :
   - Glycérine
   - Extrait de Calendula
- Agiter pendant 5 min à 600 rpm

### Exemple 4: Association Transcutol®/Labrasol®/Alcool Isopropylique

Dans cet exemple, des compositions de nettoyant auriculaire ont été formulées à partir des composants suivants : Transcutol®, Labrasol®, Alcool Isopropylique Tween 80®, Eau, Lipacide C8G®, Lipacide UG®, Trométhamine, Glycérine, Calendula.

Les % en poids de chaque composant sont indiqués en Figure 4.

Le procédé utilisé est le suivant :

### Mélange 1

- Dans un contenant adapté, chauffer l'eau à 70°C puis ajouter dans l'ordre :
   - Lipacide C8G ®
   - Lipacide UG®
   - Trométhamine
- Agiter pendant 10 min à 600 rpm
- Refroidissement de la solution jusqu'à 40 °C puis ajouter :
   - Tween 80
- Agiter pendant 5 min à 600 rpm

### Mélange 2

- Incorporer dans un contenant adapté, et dans l'ordre suivant :
   - Labrasol ®
   - Transcutol ®
   - Alcool Isopropylique
- Agiter pendant 5 min à 600 rpm
- Ajouter le mélange 1
- Agiter pendant 5 min à 600 rpm
- Ajouter dans l'ordre :
   - Glycérine
   - Extrait de Calendula
- Agiter pendant 5 min à 600 rpm

### Exemple 5: Effet céruménolytique

### Etude in vitro

L'effet céruménolytique d'un ingrédient ou d'une composition peut être démontré *in vitro* via des tests utilisant des cérumen de synthèse.

Dans la littérature, deux études *in vitro* ont été réalisées pour évaluer l'efficacité de produits céruménolytiques. L'une conduite par Nielloud et son équipe (2004) **[8],** l'autre, conduite par Sanchez (2006) **[9].** Ces études avaient pour but de déterminer l'aptitude des nettoyants auriculaires à dissoudre ou disperser le cérumen et à prévenir sa redéposition.

La composition de cérumen de synthèse, fabriqué pour chacune des deux études, était différente (tableau 1).

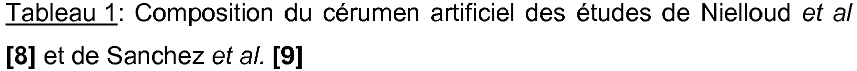

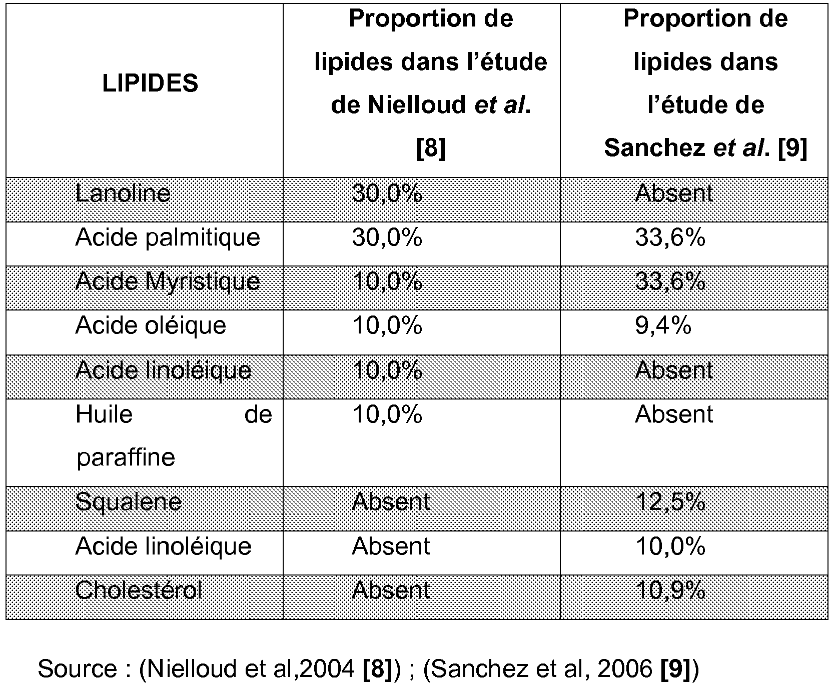

Sanchez et son équipe considèrent que le cholestérol est un lipide indispensable, car il est présent dans le cérumen de tous les chiens, or Nielloud et son équipe n'en ont pas tenu compte. De même ils soulignent que la lanoline et la paraffine ne font pas partie des lipides habituels du cérumen. Pour mémoire, le cérumen des chiens comprend chez plus de 90% des individus, du cholestérol, des esters de cholestérol, des acides gras libres, des aldéhydes gras et des cires. Par conséquent, le cérumen fabriqué par Sanchez et son équipe est celui qui s'en rapproche le plus.

Ainsi, la formule du cérumen synthétique sélectionnée permettant d'évaluer l'effet céruménolytique *in vitro* d'agents est la suivante :

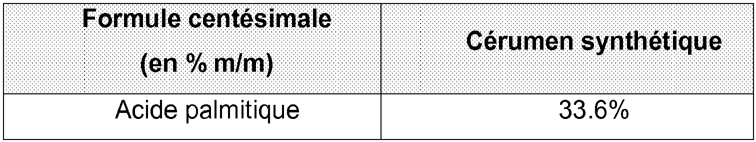

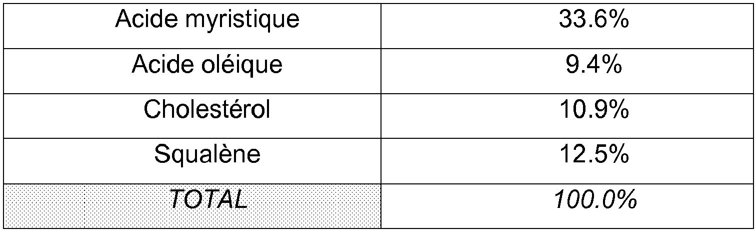

Pour sélectionner une nouvelle combinaison d'agents céruménolytiques, le test de l'effet céruménolytique a été employé en utilisant le cérumen décrit dans le tableau ci-dessus.

Lors des essais l'effet céruménolytique des ingrédients purs ou dilués a été évalué. Par la suite l'impact de l'ajout d'autres ingrédients a été étudié (effet de combinaison : effet potentialisateur) au cours de ces essais *in vitro.*

Lors de la mise au point de formule du nettoyant auriculaire les inventeurs ont constaté que :
- certains ingrédients n'ayant jamais été utilisé dans des nettoyants auriculaires, possèdent un effet céruménolytique non connu à ce jour.
- certains ingrédients n'ayant pas d'effet céruménolytique propre, peuvent potentialiser l'effet des agents céruménolytiques étudiés.

### Mode opératoire

Les modes opératoires décrits ci-après exposent les différentes étapes de la mise en œuvre du test.

### Fabrication et répartition du cérumen synthétique

Avant la mise en œuvre des essais d'évaluation de l'effet céruménolytique d'agents purs, dilués ou combinés, il est nécessaire de fabriquer du cérumen synthétique. Le mode opératoire est décrit dans le tableau suivant :

**Tableau 2:**

| **Étapes** | **Paramètres** | **Lot 140636** |
|---|---|---|
| **Mélange 1 :** Acide miristique + Acide palmitique | Vitesse d'agitation | 500 rpm |
| | Temps d'agitation | 30 min |
| | Température | 65°C |
| **Mélange 2 :** Squalène + Acide oléique | Vitesse d'agitation | 500 rpm |
| | Temps d'agitation | 5 min |
| | Température | ambiante |
| **Mélange 3 :** Mélange 2 + Cholestérol | Vitesse d'agitation | 500 rpm |
| | Temps d'agitation | 5 min |
| | Température | ambiante |
| **Mélange 4 :** Mélange 3 + Mélange 1 | Vitesse d'agitation | 500 rpm puis 1000 rpm |
| | Temps d'agitation | 25 min |
| | Température | 65°C |

Une fois la fabrication terminée, le cérumen synthétique est réparti à chaud dans des tubes à essais de 10mm x 75mm. Au fond de chaque tube est déposé 500mg de cérumen. La répartition est réalisée de manière à ce que le cérumen ne soit présent que dans le fond du tube et non sur les bords ou l'orifice, ce qui pourrait fausser les résultats.

Les tubes sont ensuite laissés au repos le temps que le cérumen se solidifie. La répartition est réalisée la veille du test *in vitro* pour laisser à minima 24h au cérumen pour refroidir et de solidifier.

### Mode opératoire du test in vitro

Suite à la fabrication du cérumen synthétique, à la préparation des agents purs ou dilués et des combinaisons, le test *in vitro* a pu donc être mis en œuvre.

Les dimensions du tube à essais, la température à laquelle a été réalisée l'essai, la quantité de cérumen synthétique ont été choisis pour représenter le plus fidèlement possible, *in vitro,* le conduit auditif du chien.

Suivant ces paramètres, le mode opératoire suivi pour mesurer l'efficacité *in vitro* d'un agent ou d'une combinaison d'agents possédant un effet céruménolytique est le suivant :
=> Le tube à essai contenant seulement le cérumen est pesé avant l'essai
=> Ensuite, 2mL de produit à tester est prélevé à l'aide d'une pipette puis est déposé au-dessus du cérumen
=> Le tube à essai est ensuite fixé à une turbine d'agitation et plongé dans un bain d'eau chauffé à 35°C (la hauteur d'eau est suffisante pour que le contenu du tube (cérumen +produit à tester) soit immergé
=> L'agitation est maintenue pendant 20 minutes à 50 rpm.
=> Après arrêt de l'agitation, le tube est retiré de la turbine du bain marie à 35°C pour ensuite être positionné à l'envers pendant 1h. Cette étape permet d'éliminer le produit et les débris de cérumen détachés par l'agent céruménolytique.
=> A la fin du temps écoulé, le tube est pesé pour évaluer la quantité de cérumen éliminé.
=> Ce processus est effectué 4 fois de suite, sur chaque même tube, pour simuler et évaluer le nombre d'application nécessaire pour éliminer le bouchon de cérumen présent dans le conduit auditif du chien.

A chaque fois, le cérumen restant dans le fond du tube est séché puis pesé. Pour l'application suivante, 2mL de produit sont à nouveau ajoutés sur le lit de cérumen restant et le déroulement des étapes décrites ci-dessus recommence.

La mise en œuvre des principales étapes de l'essai peut être schématisée comme indiqué en Figure 6.

### Mesure de l'effet céruménolytique in vitro

Comme indiqué précédemment, de manière à contrôler l'efficacité d'un agent, les tubes à essais sont pesés avant la mise en œuvre de l'essai et après la phase de repos à l'envers pendant 1h.

L'évaluation de l'élimination du cérumen est, suivant ce procédé, effectué comme pour cet exemple :
=> Masse du tube A avant l'essai 1 : 3.493g et Masse du tube A après l'essai 1 : 3.449g
=> Pourcentage de cérumen éliminé : (3.493 - 3.449)/0.5X100= 8.8% Suite à l'essai 1, la formule A utilisée dans le tube A a permis d'éliminer une partie du cérumen. Le pourcentage comme indiqué est de 8.8%

Les pourcentages sont ainsi calculés pour chaque essais : de 1 à 4. Le pourcentage total de cérumen éliminé est obtenu après avoir répété l'opération successivement 4 fois pour un même tube.

Suite aux essais céruménolytiques *in vitro* un excipient non connu pour son effet céruménolytique a été identifié comme ayant une activité céruménolytique bien marquée : l'éther monoéthylique de diéthylène glycol.

Ce dernier est utilisé comme solvant pour produit injectable ou produit à applications topiques, dermiques ou transdermiques. Comme illustré en Figure 1, une solution contenant 100 % de le transcutol® (éther monoéthylique de diéthylène glycol) possède une activité céruménolytique proche de 100%.

Il a été noté également que certains ingrédients tels que le Caprylocaproyl macrogol-8 (ou caprylcaproyl polyoxyl-8 glycéride) connu pour ses activités de surfactant et de solubilisant, peut être potentialisateur d'effet céruménolytique pour d'autres agents céruménolytiques.
Le Caprylocaproyl macrogol-8 ne possède pas d' effet céruménolytique en tant que tel. En effet les résultats du test *in vitro* montrent que son activité céruménolytique est proche de zéro (cf. Figure 1).

L'effet potentialisateur a été montré en combinaison avec l'alcool isopropylique ainsi qu'en association avec l'éther monoéthylique de diéthylène glycol.

Les résultats des tests *in vitro* montrent qu'une solution d'alcool isopropylique (100% d'alcool) possède une activité cérumunolytique de l'ordre de 60% (cf. Figure 1). Ce dernier dilué avec des produits hydrophiles par exemple 25% alcool+75% eau, voit son activité céruménolytique significativement réduite significativement pour être proche de zéro dans certains cas. En revanche, l'ajout de Caprylocaproyl macrogol-8 dans ces compositions potentialise l'effet céruménolytique de l'alcool isopropylique dilué, pour atteindre des efficacités proches de 90% (voir tableaux ci-dessous).

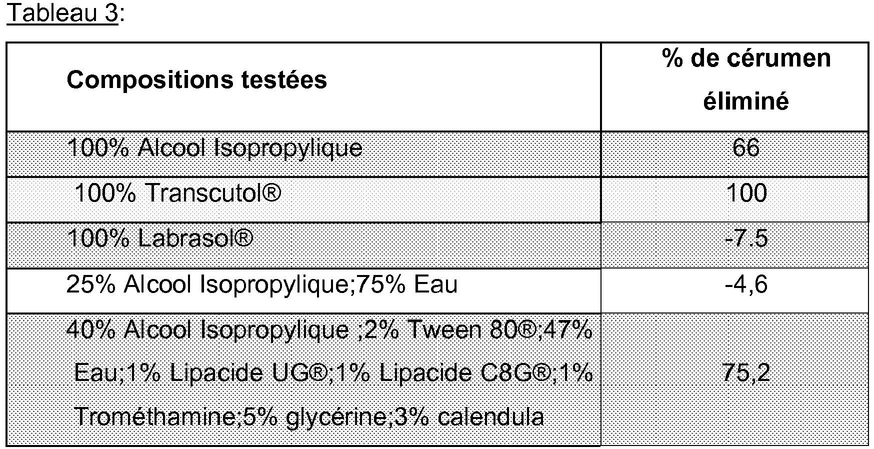

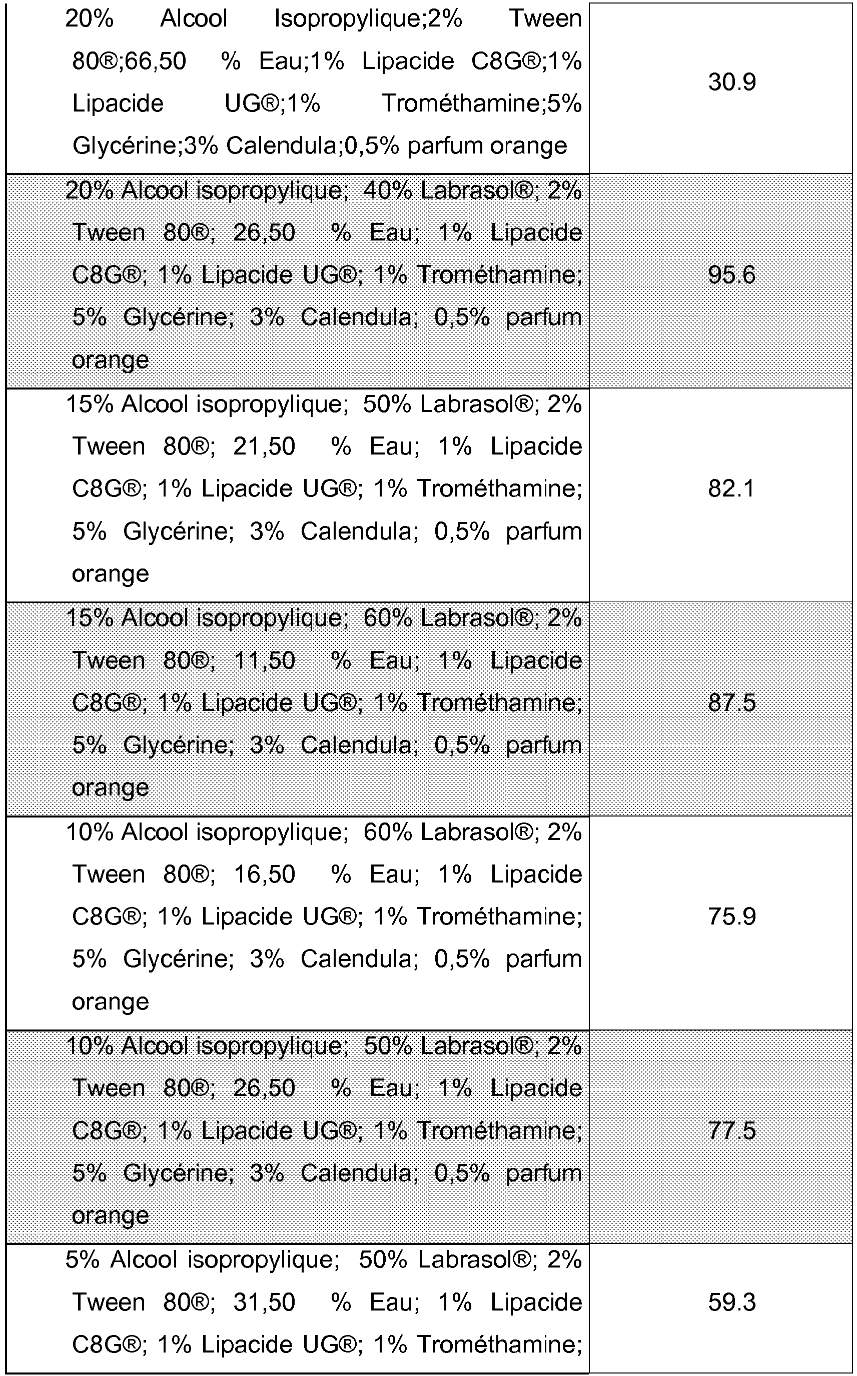

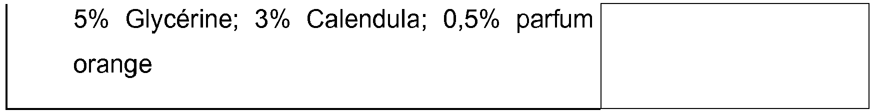

Le même phénomène est observé en combinaison avec l'éther monoéthylique de diéthylène glycol (cf. Tableau 4).

**Tableau 4:**

| | **% cérumen éliminé (après essai 4)** |
|---|---|
| 100% Transcutol® | 100,0 |
| 100% Labrasol® | -7,5 |
| 50% Transcutol®/ 50% Eau | -7,4 |
| 40% Transcutol®, 30% Labrasol®, 2% Tween 80®, 17,0 % Eau, 1% Lipacide C8G®, 1% Lipacide UG®, 1% Trométhamine, 5% Glycérine, 3% Calendula | 93.8 |
| 30% Transcutol®, 40% Labrasol®, 2% Tween 80®, 17,0 % Eau, 1% Lipacide C8G®, 1% Lipacide UG®, 1% Trométhamine, 5% Glycérine, 3% Calendula | 65.6 |
| 30% Transcutol®, 30% Labrasol®, 2% Tween 80®, 27,0 % Eau, 1% Lipacide C8G®, 1% Lipacide UG®, 1% Trométhamine, 5% Glycérine, 3% Calendula | 32.1 |
| 30% Transcutol®, 20% Labrasol®, 2% Tween 80®, 37,0 % Eau, 1% Lipacide C8G®, 1% Lipacide UG®, 1% Trométhamine, 5% Glycérine, | 17.3 |
| 3% Calendula | |

### Exemple 6: Essai céruménolytique comparatif in vivo

### Etude clinique randomisée en double aveugle et contrôlée

Une étude comparative a été réalisée pour évaluer l'efficacité nettoyante d'une composition auriculaire selon l'invention, par rapport à celle d'un produit de référence vétérinaire (Epi-Otic®), qui est un produit de référence sur le marché pour l'application visée (nettoyant auriculaire).

La composition auriculaire nettoyante selon l'invention testée dans cet exemple met en oeuvre la tri-association de l'alcool isopropylique avec l'éther monoéthylique de diéthylène glycol comme agent(s) céruménolytique (s), en combinaison avec le caprylcaproyl polyoxyl-8 glycéride.

Cette composition test a été comparée au produit référence Epi-Otic® de la société Virbac, en termes d'efficacité nettoyante du canal externe de l'oreille, sur des chiens souffrant d'otite externe spontanée. Le produit nettoyant (échantillon test ou de référence) était voué à nettoyer les parois du canal externe de l'oreille des animaux, pour préparer l'oreille à recevoir le traitement thérapeutique adapté pour soigner l'otite externe.

L'étude réalisée était une étude clinique randomisée en double aveugle et contrôlée, à savoir que les propriétaires qui ont administré le produit (produit de référence ou composition test selon l'invention) à leur chien et le vétérinaire qui a évalué les données cliniques (cytologie auriculaire et examen vidéo otoscopique) opéraient en aveugle. Les échantillons (composition selon l'invention ou Epi-Otic®) étaient identifiés par un code dont la liste et l'identité n'ont été dévoilées qu'à l'issue de l'étude clinique. Chaque échantillon a été administré de façon aléatoire suivant une liste de randomisation.

Après un examen initial du canal externe de l'oreille de l'animal, le produit nettoyant a été administré, et 30 minutes plus tard, un examen de suivi vidéo-otoscopique a été réalisé et des prélèvements pour la cytologie ont été collectés à l'aide d'écouvillons, afin d'évaluer l'efficacité nettoyante et l'effet céruménolytique des produits.

Brièvement, la quantité de cérumen présent dans le canal externe de l'oreille de chaque animal a été déterminée par vidéo-otoscopie, 30 minutes après la première application du produit nettoyant (échantillon test selon l'invention, ou échantillon de référence Epi-Otic®). La quantité de cire dans l'oreille (cérumen) a été évaluée en utilisant une feuille de score (de 0 à 4):
0: absence de cérumen
1: quelques petites plaques minces de cérumen qui adhèrent à la paroi du canal auditif
2: plusieurs plaques de cérumen de différentes épaisseurs. Seulement une légère coalescence des plaques de cérumen adhérant à la paroi du canal auditif est observée. Aucun céruminolithe
3: beaucoup de plaques épaisses de cérumen, souvent coalescentes, qui adhérent à la paroi du canal auditif. Présence possible de céruminolithes
4: la paroi du canal auditif est pratiquement complètement recouverte de cérumen

La médiane pour le score de cire d'oreille était de 6 pour les produits nettoyants utilisés immédiatement avant la première application de l'échantillon, et ce aussi bien pour les chiens recevant pour le produit test selon la présente invention, que ceux recevant le produit de référence Epi-Otic® (le score de 6 correspond à une oreille sale de départ, avant le démarrage de l'étude et toute application de produit nettoyant). Les chiens utilisés dans l'étude avaient donc tous la même base de cérumen de départ, à savoir des oreilles sales. L'évaluation à +30 minutes a montré que la médiane pour le produit test selon l'invention était de 3, alors qu'il était de 4 pour le produit de référence Epi-Otic® (cf. Tableau 5 ci-dessous). Cette différence est significative (test de Wilcoxon, p = 0,004).

**Tableau 5: Résultats des scores relatifs à la quantité de cérumen présent dans le canal externe de l'oreille des animaux**

| **Score avant application du produit nettoyant (T₀) Valeur P (p < 0,01)** | **T₀ + 30 minutes** |
|---|---|
| **Produit test selon l'invention (Médiane = 6)** | 0,001 *(Médiane*= *3)* |
| **Product de référence (Epi-Otic®) (Médiane = 6)** | 0,001 *(Médiane* = *4)* |

Les résultats de cette étude ont révélé que la composition test selon l'invention a montré une capacité de nettoyage significativement supérieure au produit de référence (Epi-Otic®). Il n'y avait pas de différence significative dans la notation cytologique entre les produits de référence (Epi-Otic®) et les échantillons test (composition selon l'invention). Toutefois, l'examen vidéo otoscopique a démontré que 30 minutes après l'administration avec l'échantillon de produit nettoyant, la quantité restante de cérumen dans le canal externe de l'oreille des animaux était significativement moins importante chez les animaux qui avaient reçu le produit test selon la présente invention.

Le produit test (composition selon l'invention) a par ailleurs été très bien toléré par tous les chiens, aucun événement indésirable majeur n'a été observé ; et l'appréciation du produit test par les propriétaires des chiens était : «très bon produit».

En résumé, le produit test (composition selon l'invention) s'est avéré être comparable au produit de référence Epi-Otic® en terme de tolérance, mais a montré une efficacité nettoyante significativement supérieure à celle d'Epi-Otic®. En effet le produit test selon la présente invention élimine une grande partie du cérumen après 30 minutes suivant la première application, ce qui n'est pas le cas d'Epi-Otic®.

### LISTE DES RÉFÉRENCES

1. HAWKE M., "Update on cerumen and ceruminolytics". Ear, Nose & Throat Journal, 2002, 81 (8 Suppl 1), p23-24.
2. MALARD O., BEAUVILLAIN DE MONTREUIL C, LEGENT F. « Pathologie acquise de l'oreille externe ». Oto-rhino-laryngologie, Editions Masson, 20-070-A10, 2005.
3. U.S. 3 422 186
4. U.S. 4 895 875
5. U.S. 5 296 472
6. U.S. 5 380 711
7. U.S. 5 480 658
8. Nielloud et al., « P-75 Development of an in vitro test to evaluate cerumen-dissolving properties of veterinary ear cleansing solutions", Veterinary Dermatology, vol 15, issue supplement s1, August 2004, p 65.
9. Sanchez et al., «In vitro investigation of cerumenolytic activity of various otic cleaners for veterinary use » Veterinary Dermatology, vol 17, N°2, April 2006, p 121-127.

## Revendications

1. Composition auriculaire nettoyante comprenant de l'alcool isopropylique et/ou de l'éther monoéthylique de diéthylène glycol en tant qu'agent(s) céruménolytique(s), éventuellement en combinaison avec du caprylcaproyl polyoxyl-8 glycéride, ladite composition comprenant :
(i) de l'éther monoéthylique de diéthylène glycol en tant qu'agent céruménolytique , en combinaison avec du caprylcaproyl polyoxyl-8 glycéride ; avec une teneur en éther monoéthylique de diéthylène glycol allant de 10 à 40% en poids, et une teneur en caprylcaproyl polyoxyl-8 glycéride allant de 20 à 60% en poids ; la teneur totale en éther monoéthylique de diéthylène glycol et caprylcaproyl polyoxyl-8 glycéride allant de 50 à 70% en poids ; les % étant exprimés par rapport au poids total de la composition ;
(ii) de l'alcool isopropylique en tant qu'agent céruménolytique, en combinaison avec du caprylcaproyl polyoxyl-8 glycéride ; avec une teneur en alcool isopropylique allant de 5 à 20% en poids, et une teneur en caprylcaproyl polyoxyl-8 glycéride allant de 20 à 60% en poids ; la teneur totale en alcool isopropylique et caprylcaproyl polyoxyl-8 glycéride allant de 35 à 75% en poids ; les % étant exprimés par rapport au poids total de la composition ;
(iii) de l'éther monoéthylique de diéthylène glycol et de l'alcool isopropylique en tant qu'agents céruménolytiques; avec une teneur en éther monoéthylique de diéthylène glycol allant de 14 à 33% en poids, et une teneur en alcool isopropylique allant de 20 à 40% en poids ; la teneur totale en éther monoéthylique de diéthylène glycol et alcool isopropylique allant de 35 à 70% en poids ; les % étant exprimés par rapport au poids total de la composition ; ou
(iv) de l'éther monoéthylique de diéthylène glycol et de l'alcool isopropylique en tant qu'agents céruménolytiques, en combinaison avec du caprylcaproyl polyoxyl-8 glycéride ;
et éventuellement un véhicule aqueux otologiquement acceptable.

2. Composition selon la revendication 1, comprenant de l'éther monoéthylique de diéthylène glycol et de l'alcool isopropylique en tant qu'agents céruménolytiques, en combinaison avec du caprylcaproyl polyoxyl-8 glycéride ; avec une teneur en éther monoéthylique de diéthylène glycol allant de 25 à 35% en poids, une teneur en alcool isopropylique allant de 5 à 10% en poids, et une teneur en caprylcaproyl polyoxyl-8 glycéride allant de 20 à 35% en poids; la teneur totale en éther monoéthylique de diéthylène glycol, alcool isopropylique et caprylcaproyl polyoxyl-8 glycéride allant de 60 à 70% en poids ; les % étant exprimés par rapport au poids total de la composition ;
et éventuellement un véhicule aqueux otologiquement acceptable.

3. Composition selon la revendication 1 ou 2, comprenant en outre un agent anti-séborrhéique, un émollient, un tensioactif, un agent tampon, un agent anti-septique, un agent anti-inflammatoire (ou agent apaisant) et/ou un conservateur.

4. Composition selon la revendication 3, dans laquelle :
- ledit agent anti-séborrhéique est choisi parmi les lipoaminoacides tels que le capryloyl glycine ou l'undécylénoyl glycine;
- ledit émollient est choisi parmi la glycérine, le propylèneglycol, le polyéthylèneglycol, la triacétine, les dérivés siliconés (ex : diméthicone), les polyols ou un mélange d'au moins deux de ceux-ci;
- ledit tensioactif est choisi parmi les polysorbates tels que le monooléate de sorbitane polyoxyéthylène, les copolymères séquencés de poly(oxyéthylène)-poly(oxypropylène), les esters de polyéthylèneglycol d'acides gras, les éthers de polyoxypropylène d'alcanes en C₁₂ à C₁₈, ou un mélange d'au moins deux de ceux-ci;
- ledit agent tampon est choisi parmi le trishydroxyméthylaminométhane, le citrate, le phosphate, le borate, l'acétate, l'hydroxyde de sodium, la glycine, la triéthanolamine, les sels de l'un quelconque de ceux-ci, ou un mélange d'au moins deux de ceux-ci;
- ledit agent anti-septique est choisi parmi un extrait de calendula (le souci), les anti-septiques de la famille des biguanides telle que la chlorhexidine, ou un mélange d'au moins deux de ceux-ci;
- ledit agent anti-inflammatoire (ou agent apaisant) est choisi parmi un extrait de calendula (le souci);
- ledit conservateur est choisi parmi l'acide borique, le dichlorure de poly [diméthylimino-2-butène-1,4-diyle]chlorure-alpha-[4-tris (2-hydroxyéthyl) ammonium], les halogénures de benzalkonium, les sels d'alexidine, les sels de chlorhexidine, ou un mélange d'au moins deux de ceux-ci.

5. Procédé non thérapeutique pour éliminer totalement ou en partie le cérumen, comprenant une étape consistant à introduire dans le canal externe de l'oreille d'un animal, une composition auriculaire nettoyante selon l'une quelconque des revendications précédentes.

6. Procédé selon la revendication 5, dans lequel l'animal est un animal domestique tel que le chien ou le chat.

7. Association d'au moins deux solvants sélectionnés parmi l'alcool isopropylique, l'éther monoéthylique de diéthylène glycol et le caprylcaproyl polyoxyl-8 glycéride pour son utilisation comme agent céruménolytique.

8. Utilisation du caprylcaproyl polyoxyl-8 glycéride en tant qu'agent potentialisateur de l'effet céruménolytique de l'alcool isopropylique et/ou de l'éther monoéthylique de diéthylène glycol d'une composition auriculaire nettoyante.

## Patentansprüche

1. Ohrenreinigungszusammensetzung, die Isopropylalkohol und/oder Diethylenglykolmonoethylether als ohrenschmalzlösende(s) Mittel umfasst, möglicherweise in Kombination mit Caprylcaproylpolyoxyl-8-glycerid, wobei die Zusammensetzung Folgendes umfasst:
(i) Diethylenglykolmonoethylether als ohrenschmalzlösendes Mittel, in Kombination mit Caprylcaproylpolyoxyl-8-glycerid; mit einem Gehalt an Diethylenglykolmonoethylether im Bereich von 10 bis 40 Gewichts-%, und einem Gehalt an Caprylcaproylpolyoxyl-8-glycerid im Bereich von 20 bis 60 Gewichts-%, wobei der Gesamtgehalt an Diethylenglykolmonoethylether und Caprylcaproylpolyoxyl-8-glycerid im Bereich von 50 bis 70 Gewichts-% liegt; wobei die %-Werte unter Bezugnahme auf das Gesamtgewicht der Zusammensetzung angegeben sind;
(ii) Isopropylalkohol als ohrenschmalzlösendes Mittel, in Kombination mit Caprylcaproylpolyoxyl-8-glycerid; mit einem Gehalt an Isopropylalkohol im Bereich von 5 bis 20 Gewichts-%, und einem Gehalt an Caprylcaproylpolyoxyl-8-glycerid im Bereich von 20 bis 60 Gewichts-%, wobei der Gesamtgehalt an Isopropylalkohol und Caprylcaproylpolyoxyl-8-glycerid im Bereich von 35 bis 75 Gewichts-% liegt; wobei die %-Werte unter Bezugnahme auf das Gesamtgewicht der Zusammensetzung angegeben sind;
(iii) Diethylenglykolmonoethylether und Isopropylalkohol als ohrenschmalzlösende Mittel; mit einem Gehalt an Diethylenglykolmonoethylether im Bereich von 14 bis 33 Gewichts-%, und einem Gehalt an Isopropylalkohol im Bereich von 20 bis 40 Gewichts-%; wobei der Gesamtgehalt an Diethylenglykolmonoethylether und Isopropylalkohol im Bereich von 35 bis 70 Gewichts-% liegt; wobei die %-Werte unter Bezugnahme auf das Gesamtgewicht der Zusammensetzung angegeben sind; oder
(iv) Diethylenglykolmonoethylether und Isopropylalkohol als ohrenschmalzlösende Mittel, in Kombination mit Caprylcaproylpolyoxyl-8-glycerid;
und möglicherweise einen ohrenheilkundlich unbedenklichen wässrigen Träger.

2. Zusammensetzung nach Anspruch 1, die Diethylenglykolmonoethylether und Isopropylalkohol als ohrenschmalzlösende Mittel umfasst, in Kombination mit Caprylcaproylpolyoxyl-8-glycerid; mit einem Gehalt an Diethylenglykolmonoethylether im Bereich von 25 bis 35 Gewichts-%, einem Gehalt an Isopropylalkohol im Bereich von 5 bis 10 Gewichts-% und einem Gehalt an Caprylcaproylpolyoxyl-8-glycerid im Bereich von 20 bis 35 Gewichts-%; wobei der Gesamtgehalt an Diethylenglykolmonoethylether, Isopropylalkohol und Caprylcaproylpolyoxyl-8-glycerid im Bereich von 60 bis 70 Gewichts-% liegt; wobei die %-Werte unter Bezugnahme auf das Gesamtgewicht der Zusammensetzung angegeben sind;
sowie möglicherweise einen ohrenheilkundlich unbedenklichen wässrigen Träger.

3. Zusammensetzung nach Anspruch 1 oder 2, wobei sie darüber hinaus ein antiseborrhoische Mittel, ein hauterweichendes Mittel, ein Tensid, einen Puffer, ein antiseptisches Mittel, ein entzündungshemmendes Mittel (oder beruhigendes Mittel) und/oder einen Konservierungsstoff umfasst.

4. Zusammensetzung nach Anspruch 3, wobei:
- das antiseborrhoische Mittel aus den Lipoaminosäuren wie etwa Capryloylglycin oder Undecylenoylglycin ausgewählt ist;
- das hauterweichende Mittel aus Glycerin, Propylenglykol, Polyethylenglykol, Triacetin, Silikonderivaten (Bsp.: Dimeticon), Polyolen, oder einer Mischung aus mindestens zweien davon ausgewählt ist;
- das Tensid aus den Polysorbaten wie etwa Polyethylenoxid-Sorbitanmonooleat, den abschnittsweise aufgebauten Copolymeren von Polyethylenoxid-Polypropylenoxid, den Polyethylenglykolestern von Fettsäuren, den Polypropylenoxidethern von C₁₂-C₁₈-Alkanen, oder einer Mischung aus mindestens zweien davon ausgewählt ist;
- der Puffer aus tris-Hydroxymethylaminomethan, Citrat, Phosphat, Borat, Acetat, Natriumhydroxid, Glycin, Triethanolamin, den Salzen eines beliebigen davon, oder einer Mischung aus mindestens zweien davon ausgewählt ist;
- das antiseptische Mittel aus einem Extrakt von Calendula (Ringelblume), Antiseptika aus der Familie der Biguanide, wie etwa Chlorhexidin, oder einer Mischung aus mindestens zweien davon ausgewählt ist;
- das entzündungshemmende Mittel (oder beruhigende Mittel) aus einem Extrakt von Calendula (Ringelblume) ausgewählt ist;
- der Konservierungsstoff aus Borsäure, Poly[dimethylimino-2-buten-1,4-diyl]chlorid-alpha-[4-tris-(2-hydroxyethyl)ammonium]dichlorid, Benzalkoniumhalogeniden, Alexidinsalzen, Chlorhexidinsalzen, oder einer Mischung aus mindestens zweien davon ausgewählt ist.

5. Nicht-therapeutisches Verfahren zur vollständigen oder teilweisen Entfernung des Ohrenschmalzes, wobei es einen Schritt umfasst, der darin besteht, eine Ohrenreinigungszusammensetzung nach einem beliebigen der vorhergehenden Ansprüche in den äußeren Gehörgang eines Tieres einzubringen.

6. Verfahren nach Anspruch 5, wobei es sich bei dem Tier um ein Haustier wie einen Hund oder eine Katze handelt.

7. Kombination aus mindestens zwei Lösungsmitteln, die aus Isopropylalkohol, Diethylenglykolmonoethylether und Caprylcaproylpolyoxyl-8-glycerid ausgewählt sind, um dieselbe als ohrenschmalzlösendes Mittel zu verwenden.

8. Verwendung von Caprylcaproylpolyoxyl-8-glycerid als Mittel zur Verstärkung der ohrenschmalzlösenden Wirkung des Isopropylalkohols und/oder des Diethylenglykolmonoethylethers einer Ohrenreinigungszusammensetzung.

## Claims

1. An auricular cleaning composition comprising isopropyl alcohol and/or diethylene glycol monoethyl ether as cerumenolytic agent(s), optionally in combination with caprylcaproyl polyoxyl-8 glyceride, said composition comprising:
(i) diethylene glycol monoethyl ether as cerumenolytic agent, in combination with caprylcaproyl polyoxyl-8 glyceride; with a diethylene glycol monoethyl ether content ranging from 10 to 40% by weight, and a caprylcaproyl polyoxyl-8 glyceride content ranging from 20 to 60% by weight; the total content of diethylene glycol monoethyl ether and caprylcaproyl polyoxyl-8 glyceride ranging from 50 to 70% by weight; the percentages being expressed with respect to the total weight of the composition;
(ii) isopropyl alcohol as cerumenolytic agent, in combination with caprylcaproyl polyoxyl-8 glyceride; with an isopropyl alcohol content ranging from 5 to 20% by weight, and a caprylcaproyl polyoxyl-8 glyceride content ranging from 20 to 60% by weight; the total content of isopropyl alcohol and caprylcaproyl polyoxyl-8 glyceride ranging from 35 to 75% by weight; the percentages being expressed with respect to the total weight of the composition;
(iii) diethylene glycol monoethyl ether and isopropyl alcohol as cerumenolytic agents; with a content of diethylene glycol monoethyl ether ranging from 14 to 33% by weight, and a content of isopropyl alcohol ranging from 20 to 40% by weight; the total content of diethylene glycol monoethyl ether and isopropyl alcohol ranging from 35 to 70% by weight; the percentages being expressed with respect to the total weight of the composition; or
(iv) diethylene glycol monoethyl ether and isopropyl alcohol as cerumenolytic agents in combination with caprylcaproyl polyoxyl-8 glyceride;
and optionally an otologically acceptable aqueous carrier.

2. A composition according to claim 1, comprising diethylene glycol monoethyl ether and isopropyl alcohol as cerumenolytic agents, in combination with caprylcaproyl polyoxyl-8 glyceride; with a diethylene glycol monoethyl ether content ranging from 25 to 35% by weight, an isopropyl alcohol content ranging from 5 to 10% by weight, and a caprylcaproyl polyoxyl-8 glyceride content ranging from 20 to 35% by weight; the total content of diethylene glycol monoethyl ether, isopropyl alcohol and caprylcaproyl polyoxyl-8 glyceride ranging from 60 to 70% by weight; the percentages being expressed with respect to the total weight of the composition;
and optionally an otologically acceptable aqueous carrier.

3. A composition according to claim 1 or 2 further comprising an anti-seborrhoeic agent, an emollient, a surfactant, a buffering agent, an antiseptic agent, an anti-inflammatory (or soothing) agent and/or a preservative.

4. A composition according to claim 3, wherein :
- said anti-seborrhoeic agent is selected from lipoamino acids such as capryloyl glycine or undecylenoyl glycine;
- said emollient is selected from glycerin, propylene glycol, polyethylene glycol, triacetin, silicone derivatives (e.g. dimethicone), polyols or a mixture of at least two of these;
- said surfactant is selected from polysorbates such as polyoxyethylene sorbitan monooleate, poly(oxyethylene)-poly(oxypropylene) block copolymers, polyethylene glycol esters of fatty acids, polyoxypropylene ethers of C12 to C18 alkanes, or a mixture of at least two of these;
- said buffering agent is selected from trishydroxymethylaminomethane, citrate, phosphate, borate, acetate, sodium hydroxide, glycine, triethanolamine, salts of any of these, or a mixture of at least two of these;
- said antiseptic agent is selected from an extract of calendula (marigold), antiseptics of the biguanide family such as chlorhexidine, or a mixture of at least two of these;
- said anti-inflammatory agent (or soothing agent) is selected from an extract of calendula (marigold);
- said preservative is selected from boric acid, poly[dimethylimino-2-butene-1,4-diyl]chloride-alpha-[4-tris (2-hydroxyethyl) ammonium] dichloride, benzalkonium halides, alexidine salts, chlorhexidine salts, or a mixture of at least two of these.

5. A non-therapeutic method for the total or partial removal of cerumen, comprising a step of introducing into the external ear canal of an animal an auricular cleaning composition according to any of the preceding claims.

6. A method according to claim 5, wherein the animal is a domestic animal such as a dog or cat.

7. Combination of at least two solvents selected from isopropyl alcohol, diethylene glycol monoethyl ether and caprylcaproyl polyoxyl-8 glyceride, for use as cerumenolytic agent.

8. Use of caprylcaproyl polyoxyl-8 glyceride as a potentiating agent for the cerumenolytic effect of isopropyl alcohol and/or diethylene glycol monoethyl ether in an auricular cleaning composition.
